(19) Europäisches Patentamt
European Patent Office
Office européen des brevets

(11) EP 1 154 974 B1

(12) EUROPÄISCHE PATENTSCHRIFT

(45) Veröffentlichungstag und Bekanntmachung des Hinweises auf die Patenterteilung:
**10.09.2003 Patentblatt 2003/37**

(21) Anmeldenummer: **00916873.3**

(22) Anmeldetag: **25.02.2000**

(51) Int Cl.[7]: **C07C 7/20**, C07C 15/46, C07B 63/04

(86) Internationale Anmeldenummer:
**PCT/EP00/01584**

(87) Internationale Veröffentlichungsnummer:
**WO 00/050367 (31.08.2000 Gazette 2000/35)**

(54) **VERFAHREN ZUR KONTINUIERLICHEN GEWINNUNG VON STYROL AUS EINEM STYROLHALTIGEN GEMISCH**

METHOD FOR THE CONTINUOUS RECOVERY OF STYRENE FROM A MIXTURE CONTAINING SAME

PROCEDE DE PREPARATION EN CONTINU DE STYRENE A PARTIR D'UN MELANGE A BASE DE STYRENE

(84) Benannte Vertragsstaaten:
**AT BE CH CY DE DK ES FI FR GB GR IE IT LI LU MC NL PT SE**

(30) Priorität: **26.02.1999 DE 19908463**

(43) Veröffentlichungstag der Anmeldung:
**21.11.2001 Patentblatt 2001/47**

(73) Patentinhaber: **BASF AKTIENGESELLSCHAFT**
**67056 Ludwigshafen (DE)**

(72) Erfinder:
- **MITULLA, Konrad**
  **D-67071 Ludwigshafen (DE)**
- **SUTORIS, Heinz, Friedrich**
  **D-67551 Worms (DE)**

(74) Vertreter: **Kinzebach, Werner, Dr. et al**
**Patentanwälte,**
**Reitstötter, Kinzebach & Partner,**
**Ludwigsplatz 4**
**67059 Ludwigshafen (DE)**

(56) Entgegenhaltungen:
**DE-A- 19 634 470**
**US-A- 4 272 344**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 1 154 974 B1

## Beschreibung

Die vorliegende Erfindung betrifft ein Verfahren zur kontinuierlichen Gewinnung von Styrol aus einem styrolhaltigen Gemisch durch Destillation in einer Kaskade mehrerer Destillationskoionnen.

Rohstyrol, d.h. ein Styrol und Ethylbenzol enthaltendes Rohgemisch wird bei der Herstellung von Styrol aus Ethylbenzol durch Dehydrierung erhalten. Das Reinstyrol wird daraus gewöhnlich durch Destillation gewonnen. Es ist bekannt, dass viele ungesättigte Verbindungen bei Temperaturerhöhung zu radikalischer Polymerisation neigen. Daher müssen vinylaromatische Verbindungen wie Styrol mit geeigneten Verbindungen stabilisiert werden, um eine vorzeitige Polymerisation bei der destillativen Reinigung der großtechnisch erhaltenen Rohprodukte zu verhindern. Üblicherweise werden diese Stabilisatoren oder Polymerisationsinhibitoren den zu destillierenden Rohprodukten vor oder während dem Reinigungsschritt zugesetzt. Trotz dieser Maßnahme erhält man einen gewissen Anteil an Oligomeren oder Polymeren. Im Einzelfall kann, besonders bei Betriebsstörungen, während der Reinigung oder Destillation eine vollständige Polymerisation der Monomercharge erfolgen. Dies führt durch den umfangreichen Reinigungsaufwand und Produktionsausfall zu hohen Kosten.

In den sowjetischen Patentschriften SU-1027150, SU-1558888 und SU-1139722 wird die Stabilisierung von Styrol durch Verwendung von Nitroxyl- oder Bis-Nitroxylverbindungen beschrieben.

Die WO-96/16921 offenbart Mischungen aus vinylaromatischen Verbindungen mit sterisch gehinderten Nitroxylverbindungen, welche durch Sauerstoffspuren aktiviert werden.

Aus der JP Hei 1-165534 sind Piperidyloxyderivate als Polymerisationsinhibitoren für Styrol bekannt.

In der US-PS-5254760 und der DE-19622498 sind Mischungen von Nitroxyl- und Nitroverbindungen zur Stabilisierung von vinylaromatischen Verbindungen während der Reinigung oder Destillation beschrieben.

Die DE 19651307 beschreibt Stoffmischungen, die vinylgruppenhaltige Verbindungen, wie Styrol sowie eine vorzeitige Polymerisation inhibierende Mischung aus einer N-Oxyl-Verbindung und einer Eisen-Verbindung enthalten. Die Mischungen sind wirksam gegen vorzeitige Polymerisation während der Reinigung oder Destillation stabilisiert.

Um eine ausreichende Stabilisierung gegen unerwünschte Polymerisation zu erzielen, werden die genannten Stabilisatoren in einer Menge von etwa 5 bis 1000 ppm, bezogen auf das styrolhaltige Gemisch, eingesetzt. Die Stabilisatoren reichern sich in der Regel im Sumpf derjenigen Kolonne an, in der das Reinstyrol über Kopf abgezogen wird. Der Destillationsrückstand, einschließlich der darin gelösten Stabilisatoren, wird in der Regel verworfen.

Die US-PS-4272344 beschreibt ein Verfahren zur Destillation vinylaromatischer Verbindungen, wobei als Polymerisationsinhibitor 2,6-Dinitro-p-kresol verwendet wird. Es ist angegeben, daß ein Teil des Destillationsrückstands in das Destillationssystem zurückgeführt werden kann, um die erforderliche Menge an Stabilisator, der während der Destillation nach und nach verbraucht wird, zu minimieren. Bei der praktischen Durchführung des Verfahrens sind die Möglichkeiten der Rückführung allerdings dadurch begrenzt, daß der Destillationsrückstand einen hohen Anteil von Styrolpolymeren enthält und daher eine hochviskose bzw. harzige Konsistenz aufweist. Die Rückführung ist daher auf sehr kleine Mengen beschränkt, damit die Konzentration an Styrolpolymeren in den Destillationskolonnen keine unannehmbar hohen Werte erreicht. Ein Großteil des 2,6-Dinitro-p-kresol-Inhibitors wird während der Destillation durch Reaktion mit den sich spontan bildenden Styrolradikalen irreversibel verbraucht. Mit der Rückführung wird lediglich der unverbrauchte Anteil des Stabilisators ausgenutzt. Eine Reaktivierung unter Neu- oder Rückbildung von Spezies, die als Radikalfänger wirksam sind, aus verbrauchtem Stabilisator findet nicht statt. Da der Restgehalt an aktivem Stabilisator im zurückgeführten Destillationsrückstand beim Verfahren nach der US-4272344 schwanken kann, ist zur Sicherstellung einer wirksamen Stabilisierung der Zusatz ausreichender Mengen frischen Stabilisators erforderlich. Insgesamt ist die Ersparnis an Stabilisator durch Rückführung bei dem Verfahren nach der US-4272344 gering.

Der vorliegenden Erfindung liegt daher die Aufgabe zugrunde, ein Verfahren zur kontinuierlichen Gewinnung von Styrol aus einem. styrolhaltigen Gemisch durch Destillation in Gegenwart eines Stabilisators anzugeben, bei dem der Stabilisator möglichst effizient genutzt wird.

Überraschenderweise wurde nun gefunden, daß N-Oxyl-Radikale, die wirksame Polymerisationsinhibitoren sind, aktivierbar oder reaktivierbar sind und in größerem Umfang in das Destillationssystem zurückgeführt werden können, als dies mit anderen Stabilisatoren möglich ist.

Gegenstand der Erfindung ist demzufolge ein Verfahren zur kontinuierlichen Gewinnung von Styrol aus einem styrolhaltigen Gemisch durch Destillation des Gemisches in einer Kaskade von n Destillationskolonnen, wobei

(i) in die erste Kolonne ein ein Stabilisatorsystem enthaltender Zulauf eingespeist wird und/oder in wenigstens eine der n-ten Destillationskolonne vorgeschalteten Destillationskolonne ein Stabilisatorsystem gegeben wird, wobei das Stabilisatorsystem N-Oxyl-Radikale umfasst;

(ii) sich im Sumpf der n-ten Destillationskolonne eine das Stabilisatorsystem enthaltende höher als Styrol siedende Hochsiederfraktion anreichert;

(iii) ein Teilstrom der Hochsiederfraktion zurückgeführt und in wenigstens eine der n-ten Destillationskolonne vorgeschalteten Destillationskolonne gegeben wird;

(iv) die Restmenge der Hochsiederfraktion aus dem Verfahren ausgeleitet wird.

Bei dem im erfindungsgemäßen Verfahren eingesetzten styrolhaltigen Gemisch handelt es sich im Allgemeinen um ein beliebiges, in der Regel großtechnisch anfallendes Produktgemisch, aus dem Styrol destillativ isoliert werden kann. Ein bevorzugtes Beispiel ist Rohstyrol, d.h. ein bei der Herstellung von Styrol aus Ethylbenzol anfallendes Rohgemisch, das neben Styrol und Ethylbenzol untergeordnete Mengen an Toluol, Benzol, Cumol und/oder α-Methylstyrol enthält. Daneben enthält Rohstyrol typischerweise bis zu 3 Gew.-%, z.B. 0,5 bis 1,2 Gew.-%, bezogen auf Styrol, Bestandteile mit einem höheren Siedepunkt als Styrol (sogenannte Höhersieder), wie Stilbene, Styrololigomere und -polymere, sowie Diphenylethan und 2-Phenylnaphthalin.

Typische Gemische, aus denen nach dem erfindungsgemäßen Verfahren Styrol gewonnen werden kann, weisen z.B. folgende Zusammensetzung auf: 1% Benzol, 2% Toluol, 40% Ethylbenzol, 56% Styrol und 1% Höhersieder.

Wegen der nahe beieinander liegenden Siedepunkte von Styrol und Ethylbenzol (145 bzw. 136°C bei atmosphärischem Druck) und der hohen Anforderungen an die Reinheit des Styrols verlangt dessen Reingewinnung einen hohen Destillationsaufwand. Die Reinigung erfolgt erfindungsgemäß durch Destillation in einer Kaskade von n Destillationskolonnen, wobei der Sumpfaustrag einer Destillationskolonne jeweils in die nachgeschaltete Destillationskolonne eingespeist wird. Die Einspeisung erfolgt vorzugsweise im Bereich der Kolonnenmitte. In die erste Kolonne wird als Zulauf das styrolhaltige Gemisch eingespeist. Der Parameter n steht für eine positive ganze Zahl $\geq 2$ und gibt die Anzahl der Destillationskolonnen in der Kaskade an. Im Allgemeinen ist bevorzugt, daß n für 2 bis 4, z.B. 2 oder 3 steht. In der n-ten Destillationskolonne wird in der Regel Reinstyrol über Kopf abgezogen, während in den der n-ten Kolonne vorgeschalteten Destillationskolonnen die leichter als Styrol siedenden Bestandteile des Rohstyrols über Kopf abgezogen werden. Der Sumpfaustrag der n-ten Kolonne kann zur Isolierung von Restmengen an Styrol und/oder Methylstyrolen einem Konzentrator, z.B. einem Dünnfilmverdampfer oder Flashverdampfer, zugeführt werden. Der dabei gewonnene Leichtsiederanteil kann in einer Aufarbeitungskolonne weiter aufgetrennt werden. Die Anordnung und Verschaltung der einzelnen Destillationskolonnen zur Durchführung des erfindungsgemäßen Verfahrens kann der Fachmann ohne weiteres aufgrund seines fachmännischen Ermessens festlegen.

Eine typische Anordnung zur technischen Destillation von Styrol ist im Kunststoff-Handbuch, Band 4 (Polystyrol), Kap. 2.3.1.4, 30 ff. (München 1996) beschrieben. Eine erfindungsgemäß verwendbare Destillationsanlage ist in Figur 1 dargestellt und kann z.B. aus einer sog. Benzol-(Toluol-)kolonne 1, der ein Gemisch aus z.B. im Wesentlichen Styrol, Ethylbenzol, Benzol und Toluol 1a zugeführt wird, einer sogenannten Ethylbenzolkolonne 2, die der Abtrennung und Rückgewinnung des Ethylbenzols 2a dient und der sogenannten Styrolkolonne 3 bestehen, aus der schließlich das Reinstyrol 3a gewonnen wird. Ethylbenzolkolonne 2 und Styrolkolonne 3 sind jeweils mit Aufkochern 2b bzw. 3b versehen, d.h. sie besitzen einen beheizbaren Sumpf.

Erfindungsgemäß wird dem Sumpf der Kolonne 3 z.B. ein Teilstrom entnommen und dem Zulauf zur Kolonne 1 und/oder 2 zugegeben. In einer bevorzugten Ausführungsform wird der Sumpfaustrag der Kolonne 3 der im Wesentlichen aus den Apparaten 4 und 5 bestehenden Einrichtung zugeführt. Dabei ist 4 ein z.B. als Dünnfilmverdampfer oder Flashverdampfer ausgebildeter Konzentrator, in dem der aus dem Kolonnensumpf 3 entnommene Produktstrom von Leichtsiedern befreit wird. Die Leichtsieder können in einer Aufarbeitungskolonne (nicht dargestellt) nochmals in Styrol und α-(β-)Methylstyrol aufgetrennt werden. Ein Teilstrom des aus 4 erhaltenen und in 5 zwischengespeicherten Konzentrats wird dann zurückgeführt.

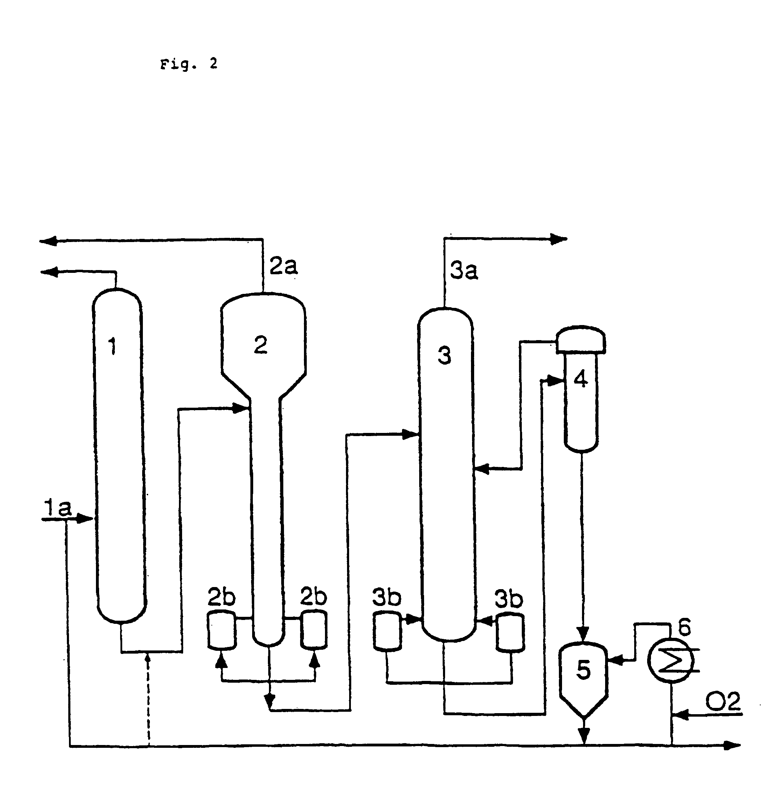

Figur 2 zeigt eine erweiterte Destillationsanlage, bei der das Konzentrat der Hochsiederfraktion gemäß einer bevorzugten Ausführungsform des erfindungsgemäßen Verfahrens mit Sauerstoff behandelt wird. Im Wärmetauscher 6 kann eine für die Aktivierung geeignete Temperatur eingestellt werden.

Erfindungsgemäß erfolgt die Destillation des styrolhaltigen Gemisches in Gegenwart eines Stabilisatorsystems, das N-Oxyl-Radikale umfasst. Es handelt sich bei den N-Oxyl-Radikalen um stabile freie Radikale, die bisweilen auch als beständige oder persistente Radikale bezeichnet werden. Sie besitzen ein oder mehrere ungepaarte Elektronen. Sie sind in der Regel als Reinsubstanz herstellbar und über Jahre unzersetzt lagerstabil. Sie sind selbst nicht in der Lage, eine radikalisch initiierte Polymerisation auszulösen. Sie fangen bereitwillig organische Radikale ab, die sich spontan z.B. bei der Destillation ethylenisch ungesättigter Verbindungen bilden. In der Regel sind die N-Oxyl-Radikale sterisch gehindert, d.h. sie leiten sich von einem sekundären Amin ab, dessen Wasserstoffatome in α-Position zum Stickstoffatom, das die Oxylgruppe trägt, sämtlich z.B. durch Alkylgruppen substituiert sind.

Das Stabilisatorsystem kann neben den N-Oxyl-Radikalen weitere Komponenten, wie die nachstehend erläuterten Polymerisationsverzögerer oder Aktivatoren, enthalten.

Geeignete N-Oxyle weisen z.B. die folgenden Strukturen auf

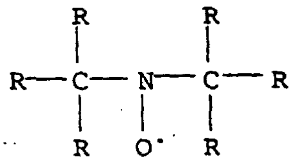

oder

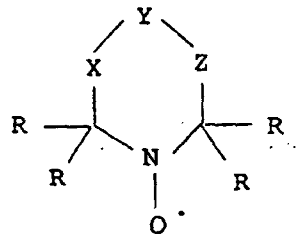

worin R für gleiche oder verschiedene Alkyl-, Cycloalkyl-, Aralkyl-oder Arylreste mit bis zu 24 C-Atomen steht, wobei geminale R-Reste auch paarweise zu einem Ringsystem verbunden sein können, und X, Y und Z unabhängig voneinander für $CR'_2$, CR'OH, CR'(COOH), O, S, CO oder eine chemische Bindung stehen, mit der Maßgabe, dass maximal ein Rest X, Y oder Z für O oder S und maximal ein Rest X, Y oder Z für eine chemische Bindung steht. R' steht für Wasserstoff oder einen Alkyl-, Cycloalkyl-, Aralkyl- oder Arylrest mit bis zu 24 C-Atomen. Beispielsweise steht R für einen $C_1$-$C_{20}$-, insbesondere $C_1$-$C_8$-Alkylrest, einen $C_5$- oder $C_6$-Cycloalkylrest, einen Benzylrest oder einen Phenylrest. X-Y-Z ist beispielsweise $-(CH_2)_2-$ oder $-(CH_2)_3-$, $-CH_2-CH(OH)-CH_2-$, $-CH_2-CO-O$oder $-CH_2-O-$.

Weiterhin kommen auch N-Oxylverbindungen mit aromatischen Substituenten wie die folgenden Strukturen in Betracht

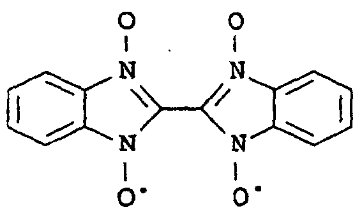

,

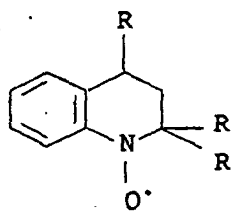

oder

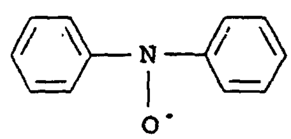

wobei die aromatischen Ringe jeweils noch 1 bis 3 inerte Substituenten tragen können, wie z.B. $C_1$-$C_4$-Alkyl, $C_1$-$C_4$-Alkoxy, Ester, Amid oder Cyano.

Vorzugsweise werden N-Oxyl-Radikale eingesetzt, die sich von cyclischen Aminen ableiten, z.B. von Piperidin- oder Pyrrolidinverbindungen, die im Ring ein weiteres Heteroatom wie Stickstoff, Sauerstoff oder Schwefel enthalten können, wobei dieses Heteroatom nicht in Nachbarstellung zum Aminstickstoff steht. Die sterische Hinderung ist durch Substituenten in beiden Nachbarstellungen zum Aminstickstoff gegeben, wobei als Substituenten Kohlenwasserstoffreste in Betracht kommen, die alle 4 Wasserstoffatome der $\alpha$-$CH_2$-Gruppen ersetzen. Beispielsweise seien als Substituenten Phenyl, $C_3$-$C_6$-Cycloalkyl, Benzyl und insbesondere $C_1$-$C_6$-Alkylreste genannt, wobei die an demselben $\alpha$-C-Atom gebundenen Alkylreste auch untereinander zu einem 5- oder 6-Ring verbunden sein können. Vorzugsweise werden als N-Oxyle sterisch gehinderter Amine Derivate des 2,2,6,6-Tetraalkylpiperidins eingesetzt.

Bevorzugte N-Oxyl-Verbindungen sind solche der allgemeinen Formel (II) oder (II')

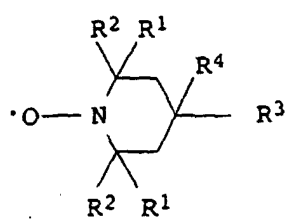

(II),

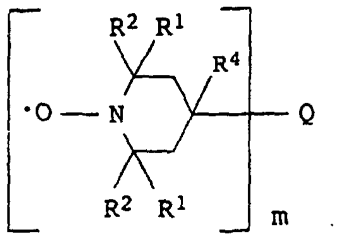

(II')

wobei

R¹ und R² → $R^1$ und $R^2$ unabhängig voneinander jeweils $C_1$-$C_4$-Alkyl, Phenyl oder $R^1$ und $R^2$ gemeinsam mit dem C-Atom, an das sie gebunden sind, einen 5- oder 6-gliedrigen, gegebenenfalls substituierten, gesättigten Kohlenwasserstoffring, der 1 oder 2 Heteroatome, ausgewählt unter O, S oder N, sowie 1 oder 2 Ketogruppen enthalten kann,

$R^3$ Wasserstoff, Hydroxy, Amino, $SO_3H$, $SO_3M$, $PO_3H_2$, $PO_3HM$, $PO_3M_2$, siliciumorganische Reste oder einen einwertigen über Kohlenstoff, Sauerstoff oder Stickstoff gebundenen organischen Rest mit vorzugsweise 1 bis 36 Atomen, wobei M für ein Alkalimetall, vorzugsweise Li, Na und K, steht,

$R^4$ Wasserstoff, $C_1$-$C_{12}$-Alkyl, $C_1$-$C_{12}$-Alkoxy

oder $R^3$ und $R^4$ gemeinsam Sauerstoff
oder $R^3$ und $R^4$ gemeinsam mit dem C-Atom, an das sie gebunden sind, einen 5- oder 6-gliedrigen, gegebenenfalls substituierten, gesättigten Ring bilden, der 1 oder 2 Heteroatome, ausgewählt unter O, S oder N, sowie 1 oder 2 Ketogruppen enthalten kann,

Q einen m-wertigen über Kohlenstoff, Sauerstoff oder Stickstoff gebundenen organischen Rest mit vorzugsweise 2 bis 10 000 Atomen, insbesondere 4 bis 2000 Atomen,

m 2 bis 100, vorzugsweise 2 oder 3,

bedeuten.

$R^1$ und $R^2$ können $C_1$-$C_4$-Alkylgruppen, wie Methyl, Ethyl, n-Propyl, iso-Propyl, n-Butyl, iso-Butyl, sec-Butyl oder tert.-Butyl sein oder sie können zusammen eine Tetra- oder Pentamethylengruppe bilden. Vorzugsweise sind $R^1$ und $R^2$ Methylgruppen.

Als $R^4$ kommen beispielsweise Wasserstoff, die oben genannten $C_1$-$C_4$-Alkylgruppen sowie Pentyl, sec-Pentyl, tert-Pentyl, Neopentyl, 2,3-Dimethyl-but-2-yl, Hexyl, 2-Methylpentyl, Heptyl, 2-Methylhexyl, 2-Ethylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl in Betracht.

Bevorzugte Reste $R^3$ sind Wasserstoff,

$C_1$-$C_{20}$-Alkylgruppen, wie Methyl, Ethyl, n-Propyl, iso-Propyl, n-Butyl, iso-Butyl, Pentyl,

Hydroxy,

$C_2$-$C_{20}$-Alkoxygruppen, wie Methoxy, Ethoxy, Propoxy, und t-Butoxy,

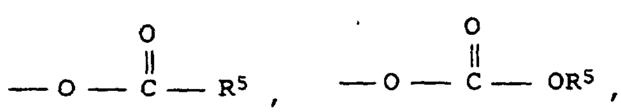

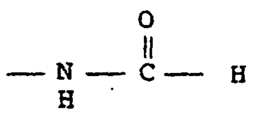, 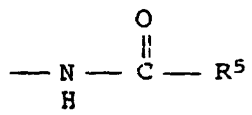, 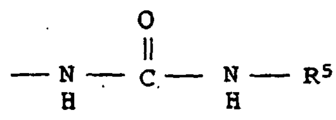,

wobei $R^5$ $C_1$-$C_{12}$-Alkyl, $C_6$-$C_{12}$-Aryl oder $C_7$-$C_{14}$-Aralkyl bedeutet,

sowie siliciumorganische Reste der Formel

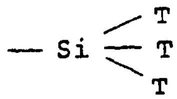

wobei die Gruppen T gleich oder verschieden voneinander sein können und $C_1$-$C_{12}$-Alkyl oder Phenyl bedeuten.

Beispiele solcher siliciumorganischer Reste sind $-Si(CH_3)_3$ und $-Si(C_2H_5)_3$.

$R^3$ und $R^4$ können gemeinsam mit dem C-Atom, an das sie gebunden sind, z.B. für

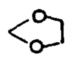

stehen.

Bevorzugte Reste Q sind beispielsweise die folgenden Reste

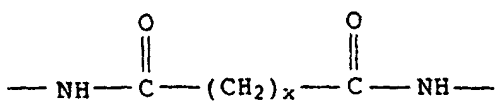,

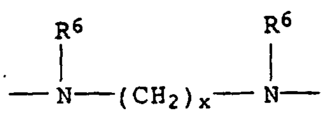, 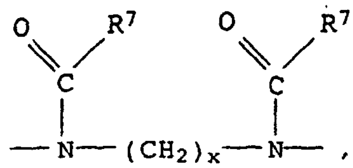,

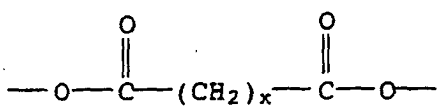, 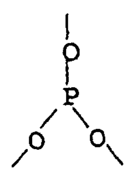

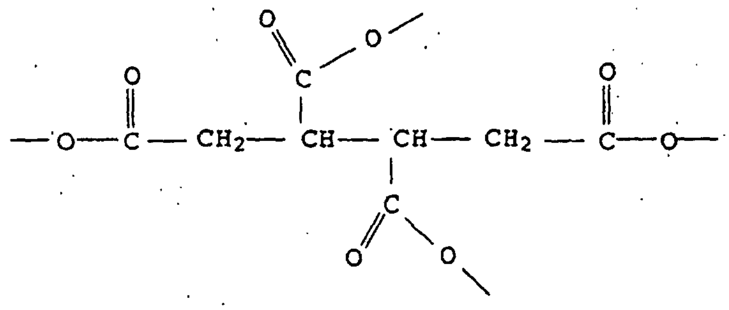

wobei

$R^6$ $C_1$-$C_{12}$-Alkyl,

$R^7$ Wasserstoff oder $C_1$-$C_{18}$-Alkyl,

x 1 bis 12

bedeuten.

Weitere geeignete N-Oxyle sind auch oligomere oder polymere Verbindungen, welche als Polymerhauptkette ein Polysiloxan besitzen und in der Seitenkette mit N-Oxyl-Gruppierungen.substituiert sind, welche sich vom 2,2,6,6-Tetraalkylpiperidin ableiten. Als bevorzugte N-Oxylgruppierung wird dabei der 2,2,6,6-Tetramethylpiperidin-N-oxyl-Rest verwendet. Beispiele für solche erfindungsgemäß ebenfalls einzusetzende N-Oxyle finden sich in der WO 69/17002. Weiter sind in dieser Druckschrift Beispiele für Synthesen der den N-Oxylen zugrundeliegenden Aminoverbindungen aufgeführt.

Weitere erfindungsgemäß geeignete N-Oxyl-Radikale sind die in der DE 19651307 als Bestandteil der dort offenbarten Stoffmischung genannten N-Oxyl-Radikale. Auf diese Druckschrift wird vollinhaltlich Bezug genommen.

Bevorzugte Nitroxylverbindungen sind die folgenden: 1-Oxyl-2,2,6,6-tetramethylpiperidin, 1-Oxyl-2,2,6,6-tetramethylpiperidin-4-ol, 1-Oxyl-2,2,6,6-tetramethylpiperidin-4-on, 1-Oxyl-2,2,6,6-tetramethylpiperidin-4-yl-acetat, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-oxyl-2,2,6,6-tetramethylpiperidin-4-yl)-succinat, Bis(1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,6,6-tetramethylpiperidin-4-yl)-phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexyhydroterephthalat, N,N'-Bis(1-oxyl-2,2,6,6-tetramethylpiperidin-4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-oxyl-2,2,6,6,-tetramethylpiperidin-4-yl]-s-triazin, N,N'-Bis(1-oxyl-2,2,6,6-tetramethylpiperidin-4-yl)-N,N'-bisformyl-1,6-diaminohexan, 4,4'-Ethylenbis(1-oxyl-2,2,6,6-tetramethylpiperazin-3-on) und Tris-(2,2,6,6-tetramethyl-1-oxyl-piperidin-4-yl)phosphit.

Die Herstellung der erfindungsgemäß verwendeten N-Oxyl-Radikale ist über verschiedene an sich bekannte Syntheseschritte möglich. Eine bevorzugte Herstellungsweise bedient sich der Oxidation eines sekundären Amins, dessen NH-Gruppe oxidativ in die entsprechende N-Oxylgruppe überführt wird. Als Oxidationsmittel kommen Peroxide, wie $H_2O_2$, t-Butylhydroperoxid, Cumolhydroperoxid, Persäuren, wie Metachlorperbenzoesäure, $\alpha$-Chlorperbenzoesäure, Peressigsäure, Paranitroperbenzoesäure, Perbenzoesäure oder Magnesiummonoperoxyphthalat in Betracht.. Die Oxidation kann in einem inerten Lösungsmittel, wie $CH_2Cl_2$, Petrolether, Toluol, Xylol oder Benzol, erfolgen.

Die zugrundeliegenden sekundären Amine sind entweder literaturbekannt oder können vom Fachmann der organisch chemischen Synthese durch Abwandlung an sich bekannter Verfahren ohne weiteres hergestellt werden. Die DE 19651307 offenbart die Herstellung verschiedener erfindungsgemäß geeigneter N-Oxyl-Radikale.

In die erste Destillationskolonne wird als Zulauf das styrolhaltige Gemisch eingespeist. Dieses kann bereits mit einem N-Oxyl-Radikale umfassenden Stabilisatorsystem versetzt sein. Dieser Fall liegt regelmäßig vor, wenn z.B. bei der Herstellung des styrolhaltigen Gemisches ein styrolhaltiges gasförmiges Reaktionsgemisch mit einem stabilisatorhaltigen flüssigen Rohgemisch kondensiert wird. Alternativ oder zusätzlich kann das Stabilisatorsystem in wenigstens eine der n-ten Destillationskolonne vorgeschalteten Destillationskolonne gegeben werden. Das Stabilisatorsystem kann zweckmäßigerweise dem Zulauf einer Destillationskolonne beigemischt werden oder aber in den Sumpf der Kolonne gegeben werden.

Bei den N-Oxyl-Radikalen und den fakultativen Komponenten des Stabilisatorsystems handelt es sich um

schwerflüchtige Verbindungen. Im Sumpf der n-ten Destillationskolonne reichert sich daher eine Hochsiederfraktion an, die das Stabilisatorsystem enthält. In der Regel besteht die Hochsiederfraktion aus dem höhersiedenden Bestandteilen des styrolhaltigen Gemisches und/oder Styrololigomeren, die sich in geringem Umfang bei der Destillation bilden. In Einzelfällen kann dem styrolhaltigen Gemisch vor der Destillation darüberhinaus ein höher als Styrol siedendes Lösungsmittel zugemischt werden, das sich in der Hochsiederfraktion anreichert und als Träger für das Stabilisatorsystem dient.

Erfindungsgemäß wird ein Teilstrom der sich im Sumpf der n-ten Destiilationskolonne anreichernden Lösung des Stabilisatorsystems in der Hochsiederfraktion zurückgeführt und zum Zulauf zu wenigstens einer der n-ten Destillationskolonne vorgeschalteten Destillationkolonne gegeben. Der zurückgeführte Strom kann aufgeteilt und an mehreren Punkten zugegeben werden, z.B. zum Zulauf der ersten und zum Zulauf der zweiten Kolonne. Für den bevorzugten Fall einer Destillation in drei aufeinanderfolgenden Destillationskolonnen werden vorzugsweise 50 bis 100 Gew.-% der zurückgeführten Hochsiederfraktion zu dem Zulauf der ersten Destillationskolonne gegeben und 0 bis 50 Gew.-% der zurückgeführten Hochsiederfraktion zu dem Zulauf der zweiten Destillationskolonne. Zweckmäßigerweise wird die zurückgeführte Stabilisatorlösung dem Zulauf einer vorgeschalteten Destillationskolonne beigemischt; die zurückgeführte Lösung kann aber auch direkt in den Sumpf einer vorgeschalteten Destillationskolonne gegeben werden.

In der Regel ist es bevorzugt, die dem Sumpf der n-ten Destillationskolonne entnommene Hochsiederfraktion vor der Rückführung bzw. dem Ausleiten aufzukonzentrieren, d.h. von Leichtsiedern zu befreien. Hierzu sind beispielsweise Vorrichtungen, wie ein Dünnfilmverdampfer oder Flashverdampfer geeignet. Die dabei gewonnene Leichtsiederfraktion kann in einer Aufarbeitungskolonne nochmals in Styrol und $\alpha$- oder $\beta$-Methylstyrol aufgetrennt werden. Es ist bevorzugt, daß der Gehalt an $\alpha$-Methylstyrol in der zurückgeführten Stabilisatorlösung weniger als 3 Gew.-%, z.B. 0,01 bis 2 Gew.-%, beträgt. Höhere Anteile an $\alpha$-Methylstyrol in der zurückgeführten Stabilisatorlösung können unter Umständen dazu führen, dass der $\alpha$-Methylstyrol-Anteil in der in der n-ten Destillationskolonne über Kopf abgezogenen Reinstyrolfraktion unerwünschterweise ansteigt. Durch eine vorstehend geschilderte Aufkonzentrierung der Hochsiederfraktion vor der Rückführung kann eine Senkung des $\alpha$-Methylstyrol-Anteils auf die angegebenen Werte ohne weiteres erreicht werden. Nach der Konzentrierung beträgt die Konzentration der N-Oxyl-Radikale in der Hochsiederfraktion im Allgemeinen 0,2 bis 100 g/l.

Mit dem Zulauf zur ersten Kolonne oder durch Zugabe in eine Kolonne wird absatzweise oder kontinuierlich eine frische Menge an N-Oxyl-Radikale umfassendem Stabilisatorsystem ergänzt, um die Menge an Stabilisatorsystem zu ersetzen, die mit der ausgeleiteten Teilmenge der Hochsiederfraktion aus dem Sumpf der n-ten Destillationskolonne dem System entzogen werden. Die ergänzte Menge an N-Oxyl-Radikalen und gegebenenfalls weiterer Komponenten des Stabilisatorsystems kann in Substanz oder in Form einer Lösung in einem Lösungsmittel, wie Wasser, $C_1$-$C_6$-Alkanolen, wie Methanol, Ethanol, Propanol sowie n-, i-, t-Butanol, gegebenenfalls in Mischung mit Wasser, Ketone, wie Aceton, Methylethylketon, Methylpropylketon, Methylbutylketon, Diolen, wie Glycol oder Propylenglycol, sowie deren Alkylmono- und -diether, oligomere oder polymere Ethylenglycole und Propylenglycole sowie deren Alkylether, Diamine, wie Ethylendiamin oder Propylendiamin sowie deren Alkylmono- oder -diiminoether, oligomere oder polymere Ethylendiamine sowie deren Alkyliminoether, zugegeben werden. Vorzugsweise wird jedoch das zu reinigende styrolhaltige Gemisch als Lösungs- oder Suspendiermittel für das Stabilisatorsystem verwendet. So kann das bei der Dehydrierung von Ethylbenzol anfallende Gemisch, welches überwiegend aus Styrol, Ethylbenzol, Toluol sowie weiteren substituierten Aromaten besteht, zu diesem Zweck eingesetzt werden. Zweckmäßigerweise wird die Lösung des Stabilisatorsystems zum Zulauf einer der n-ten Destillationskolonne vorgeschalteten Destillationskolonne gegeben, z.B. diesem beigemischt. So kann man z.B. zum Zulauf der ersten und/oder zweiten Destillationskolonne eine kontinuierliche Zudosierung frischer N-Oxyl-Radikal-Lösung vorsehen.

Die N-Oxyl-Radikale werden vorzugsweise in solcher Menge eingesetzt, daß die Konzentration der N-Oxyl-Radikale im Sumpf jeder Destillationskolonne wenigstens 0,1 ppm, insbesondere 1 bis 500, vorzugsweise 5 bis 150 ppm beträgt. Die Menge im Sumpf einer Destillationskolonne setzt sich aus der zurückgeführten und frisch zugegebenen Menge an N-Oxyl-Radikal zusammen.

Die erfindungsgemäß verwendeten N-Oxyl-Radikale sind wirksame Inhibitoren der Styrolpolymerisation und drängen die Bildung von Styrolpolymeren während der Destillation stark zurück. Die Hochsiederfraktion in der n-ten Kolonne weist daher - auch nach einer Aufkonzentrierung - eine wünschenswert niedrige Viskosität auf, so daß problemlos größere Teilmengen zurückgeführt werden können. Im Sumpf der n-ten Destillationskolonne herrscht in der Regel eine höhere Temperatur als in den Sümpfen der vorgeschalteten Kolonnen, da in den vorgeschalteten Kolonnen niedriger als Styrol siedende Fraktionen abdestilliert werden, während in der n-ten Kolonne Styrol über Kopf abgezogen wird. Es wird angenommen, daß im Sumpf der n-ten Destillationskolonne eine teilweise Reaktivierung der N-Oxyl-Radikale stattfindet. Die Reaktivierung kann durch das folgende Formelschema veranschaulicht werden:

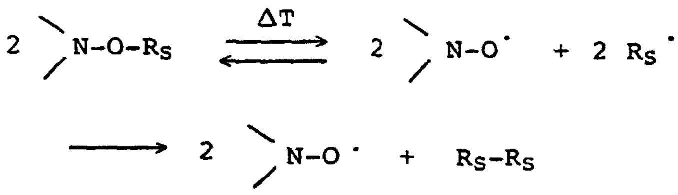


worin $R_S$ für einen einen oder mehrere Styrolreste umfassenden organischen Rest steht. Die Bindung des Restes $R_S$ zum Sauerstoffatom des Nitroxylradikals ist bei erhöhter Temperatur reversibel. Bei Temperaturerhöhung liegt in einer Gleichgewichtsreaktion eine stationäre Konzentration freier $R_S$-Radikale vor, die paarweise kombinieren können, wobei die Nitroxylradikale wieder freigesetzt werden.

Als Maß für die erfindungsgemäße Rückführung von N-Oxyl-Radikalen, die im zurückgeführten Strom der Hochsiederfraktion enthalten sind, läßt sich die Zahl der Zyklen Z definieren, die die N-Oxyl-Radikale die (n-1)-te Destillationskolonne im Durchschnitt durchlaufen. Die Zyklenzahl Z ist über die folgende Gleichung mit dem Anteil x der zurückgeführten Menge Hochsiederfraktion, bezogen auf die Gesamtmenge an Hochsiederfraktion, die im Sumpf der n-ten Destillationskolonne anfällt, verknüpft:

$$Z = \frac{1}{1-x}$$

Vorzugsweise durchlaufen die N-Oxyl-Radikale die (n-1)-te Destillationskolonne im Durchschnitt mindestens 1,4 mal, vorzugsweise 2,0 mal, insbesondere 2,5 mal, besonders bevorzugt 3 mal. Mit den genannten Zyklenzahlen korrespondieren im Allgemeinen Anteile von mehr als 0,3, vorzugsweise mehr als 0,5, insbesondere mehr als 0,6, besonders bevorzugt mehr als 0,67, der zurückgeführten Stabilisatorlösung. Im Allgemeinen ist die Rückführung von 10 bis 90 Gew.-%, vorzugsweise 30 bis 85 Gew.-%, insbesondere 50 bis 80 Gew.-%, der im Sumpf der n-ten Destillationskolonne anfallenden Hochsiederfraktion bevorzugt.

Es wurde anhand von ESR-Untersuchungen gefunden, daß eine besonders gute Reaktivierung der zurückgeführten Stabilisatorlösung erreicht werden kann, wenn der Teilstrom vor der Rückführung auf eine Temperatur von mehr als 130°C erwärmt wird. In einer bevorzugten Ausführungsform des erfindungsgemäßen Verfahrens wird der Teilstrom der Lösung des Stabilisatorsystems vor der Rückführung daher auf eine Temperatur von mehr als 130°C, insbesondere 135-160°C, erwärmt. Das Erwärmen kann zweckmäßigerweise über eine Dauer von 1 bis 300 min, vorzugsweise 10 bis 60 min, durchgeführt werden.

In einer weiteren bevorzugten Ausführungsform des erfindungsgemäßen Verfahrens enthält das Stabilisatorsystem ferner wenigstens einen Polymerisationsverzögerer. Als Polymerisationsverzögerer werden Substanzen bezeichnet, die eine radikalisch initiierte Polymerisation der Styrolmonomeren nicht vollständig unterbinden, sondern die Polymerisationsgeschwindigkeit herabsetzen. Die Kombination der erfindungsgemäß zu verwendenden N-Oxyl-Radikale mit wenigstens einem Polymerisationsverzögerer weist den Vorteil auf, daß, etwa im Falle einer Betriebsstörung, wenn die Konzentration an N-Oxyl-Radikalen unterhalb eines für eine wirksame Inhibierung erforderlichen Schwellenwert sinkt, keine schlagartig einsetzende Polymerisation der im System befindlichen Monomerenmenge erfolgt. Vielmehr findet ein langsamer Anstieg des Oligomer- bzw. Polymergehalts statt, so daß erforderlichenfalls Gegenmaßnahmen ergriffen werden können. Die Kombination der N-Oxyl-Radikale mit einem Polymerisationsverzögerer weist ferner einen synergistischen Effekt auf. Dieser ist darin zu sehen, daß sich die unterschiedlichen Wirkungsmechanismen ergänzen, wobei bei gleicher Gesamtkonzentration des Stabilisatorsystems bei Verwendung einer Kombination von N-Oxyl-Radikalen mit einem Polymerisationsverzögerer eine höhere polymerisationsinhibierende Wirkung erreicht wird als bei isolierter Verwendung von N-Oxyl-Radikalen oder Polymerisationsverzögerern. vorzugsweise wird der Polymerisationsverzögerer in einer Menge von 50 bis 2000 ppm, bezogen auf Styrol, eingesetzt. Das Gewichtsverhältnis von N-Oxyl-Radikalen zu Polymerisationsverzögerer liegt vorzugsweise in einem Bereich von 1:20 bis 20:1.

Als Polymerisationsverzögerer sind insbesondere aromatische Nitroverbindungen, insbesondere der Formel III geeignet

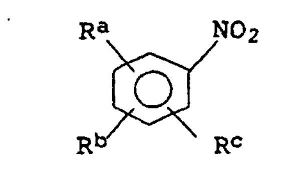

(III),

worin

$R^a$, $R^b$ und $R^c$ unabhängig voneinander jeweils Wasserstoff, $C_1$-$C_6$-Alkyl, Halogen oder einen Rest der Formel CN, SCN, NCO, OH, $NO_2$, COOH, CHO, $SO_2H$ oder $SO_3H$ bedeuten,

wobei der aromatische Ring benzoanelliert sein kann.

In Frage kommende Verbindungen sind beispielsweise 1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol oder 3-Iod-4-cyano-5-nitrophenol. Bevorzugt werden aromatische Nitroverbindungen, wie 2,6-Dinitro-4-methylphenol, 2-Nitro-4-methylphenol, 2,4-Dinitro-6-sec-butylphenol bzw. 2,4-Dinitro-6-methylphenol verwendet.

Das Stabilisatorsystem im erfindungsgemäßen Verfahren kann gegebenenfalls noch einen oder mehrere Costabilisatoren aus der Gruppe der aromatischen Nitrosoverbindungen, Phenothiazine, Chinone, Hydrochinone und deren Ether, Phenole und deren Ether, Hydroxylamine und Phenylendiamine, enthalten.

Weitere Costabilisatoren können auch substituierte Phenole oder Hydrochinone, beispielsweise die folgenden: 4-tert-Butylbrenzcatechin, Methoxyhydrochinon, 2,6-Di-tert-butyl-4-methylphenol, n-Octadecyl-ß-(3,5-di-tert-butyl-4-hydroxyphenyl)-propionat, 1,1,3-Tris-(2-methyl-4-hydroxy-5-tert-butylphenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-isocyanurat, 1,3,5-Tris-[β-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrit-tetrakis-[β-(3,5-di-tert-butyl-4-hydroxy-phenyl)-propionat] sein.

In einer bevorzugten Ausführungsform des erfindungsgemäßen Verfahrens enthält das Stabilisatorsystem neben den erfindungsgemäß verwendeten N-Oxyl-Radikalen ferner einen Aktivator. Als Aktivator wird eine chemische Verbindung bezeichnet, die die Wirkung der N-Oxyl-Radikale steigern kann, indem sie z.B. Radikalkombinationsreaktionen katalysiert.

Vorzugsweise wird der Aktivator in einer Menge von 0,01 bis 20 Gew.-%, bezogen auf die N-Oxyl-Radikale, eingesetzt.

Als Aktivatoren sind insbesondere Eisenverbindungen oder andere Übergangsmetallverbindungen geeignet, insbesondere solche, die in verschiedenen Wertigkeitsstufen vorliegen können.

Bevorzugte als Aktivatoren geeignete Eisenverbindungen sind ausgewählt aus der Gruppe der

a) Eisencarbonyle und Carbonylferrate,
b) metallorganischen Eisencarbonylverbindungen,
c) unsubstituierten und substituierten Ferrocen-Verbindungen,
d) Eisenverbindungen mit Liganden, welche als Donoratome alleine oder in Mischung Sauerstoff, Stickstoff, Schwefel oder Phosphor enthalten,
e) Eisenhalogenid- und Eisenpseudohalogenidverbindungen.

Unter die Gruppe a) fallen beispielsweise Verbindungen wie Eisenpentacarbonyl, $Fe(CO)_5$, Dieisennonacarbonyl, $Fe_2(CO)_9$, Trieisendodecacarbonyl, $Fe_3(CO)_{12}$, oder Hexaeisenoctadecacarbonyl, $Fe_6(CO)_{18}$, welche durchweg in wenig polaren oder unpolaren Medien löslich sind. Weiter sind hier zu nennen die Carbonylferrate wie $M_2Fe(CO)_4$, $M_2Fe_2(CO)_8$ und $M_2Fe_3(CO)_{11}$, wobei M für ein Äquivalent eines Alkali- oder Erdalkalimetalls steht. Vorzugsweise werden die entsprechenden Na-Verbindungen eingesetzt.

Metallorganische Eisencarbonylverbindungen der Gruppe b) sind beispielsweise Verbindungen der Formel

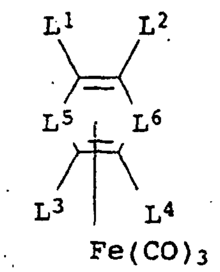

oder

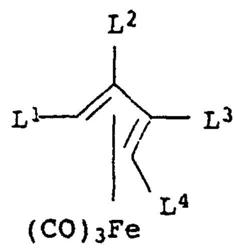

wobei die Variablen bedeuten

$L^1$ - $L^4$ Wasserstoff, $C_1$-$C_4$-Alkyle wie Methyl, Ethyl, Propyl oder t-Butyl

$L^5$, $L^6$ $-(CH_2)_n-$ oder -CO-, wobei für die Variablen $L^5$ und $L^6$ n unabhängig voneinander 0, 1, 2 oder 3 bedeutet.

Geeignete Verbindungen sind z. B.

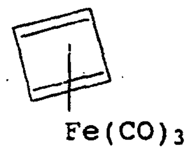 , 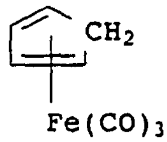 , 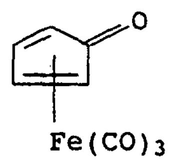 , 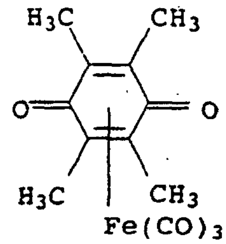

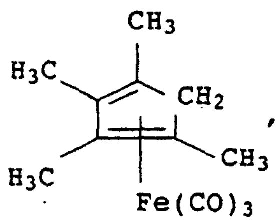 , 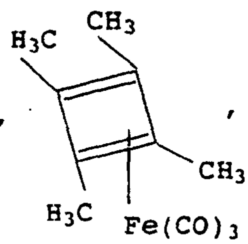 , 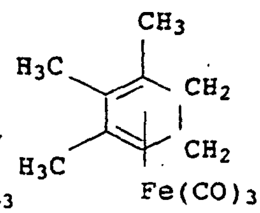 , 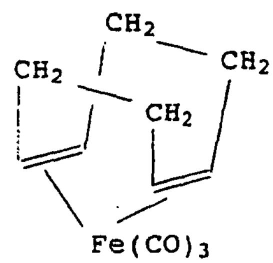

sowie

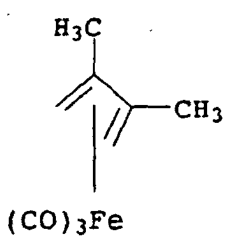 , 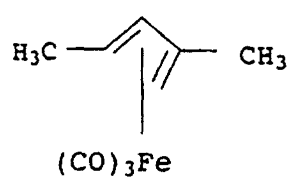 , 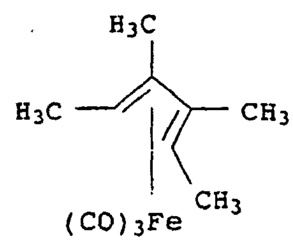

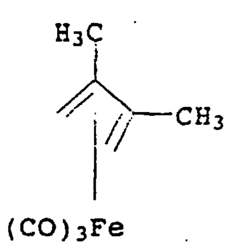 , 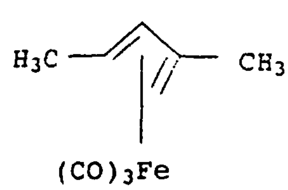 , 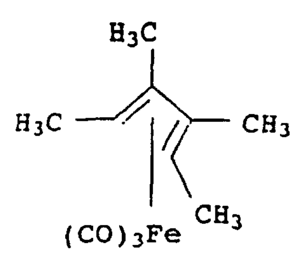

Weiter können aus dieser Gruppe erfindungsgemäß auch zweikernige Fe-Verbindungen wie $[H_5C_5Fe(CO)_2]_2$, $[(H_3C)_5C_5Fe(CO)_2]_2$ sowie die sich daraus ableitenden Ferrate $M[Fe(CO)_2C_5H_5]$ und $M[Fe(CO)_2(H_3C)_5C_5]$ verwendet werden, wobei auch hier M für ein Äquivalent eines Alkali- oder Erdalkalimetalls steht und bevorzugt die entsprechenden Na-Verbindungen Einsatz finden.

Zu den erfindungsgemäß einzusetzenden Verbindungen der Gruppe c) zählen das Ferrocen selbst sowie die an einem oder beiden Cyclopentadienylringen substituierten Derivate. Weiter können auch dimere Ferrocenderiyate

eingesetzt werden. Die Verknüpfung der einzelnen Ferroceneinheiten erfolgt dabei über je ein C-Atom des Cyclopentadienylringes durch eine chemische Bindung oder eine Methylen-, Ethylen-, Propylen-, Butylen- oder Phenylphosphin-Brücke.

Als Substituenten der Cyclopentadienylringe kommen in Frage $C_1$-$C_4$-Alkenylreste, $C_7$-$C_{10}$-Aroyl, $C_1$-$C_4$-Alkylreste wie Methyl, Ethyl, n-Propyl, i-Propyl, n-Butyl, i-Butyl, sec.-Butyl oder t-Butyl. Weiter können in diesen Substituenten ein oder zwei $CH_2$oder $CH_3$-Gruppen ersetzt sein durch O, NH, $NCH_3$ oder OH, $NH_2$. Die Bindung dieser Heteroatome oder heteroatomhaltigen Fragmente erfolgt dabei zu C-Atomen. Es können weiterhin auch ein oder zwei $CH_2$-Gruppen durch CO oder ein oder zwei $CH_3$-Gruppen durch CN ersetzt sein. Daneben können auch, gegebenenfalls neben den bereits erwähnten Gruppen, Diphenylphosphinreste als Substituenten an den Cyclopentadienylringen fungieren.

Beispiele für erfindungsgemäß einzusetzende Ferrocenderivate sind

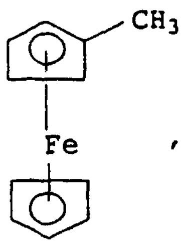 , 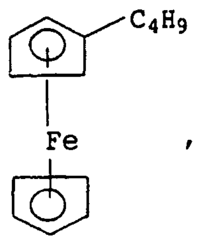 , 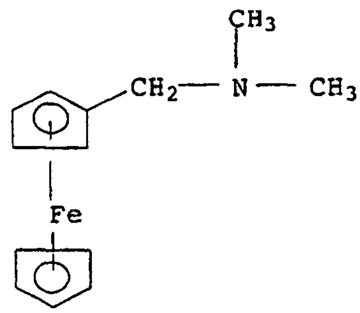 ,

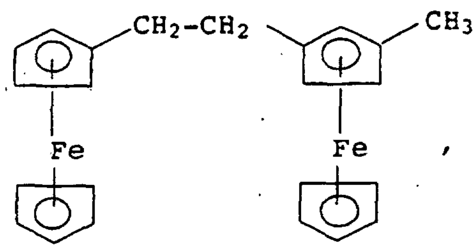 , 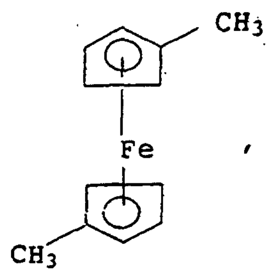 ,

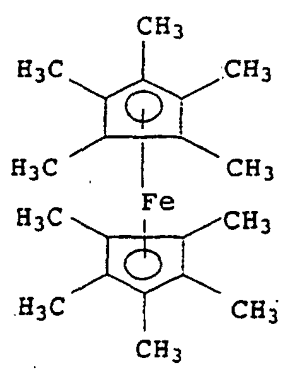

Als Verbindungen der Gruppe d) können beispielsweise eingesetzt werden Komplexe oder Salze des Fe(II) /Fe(III) mit O-haltigen Liganden wie Sulfat, Acetat, Oxalat, Citrat, Tartrat, Lactat, Gluconat oder Acetylacetonat (acac), d.h. Verbindungen wie

$$[Fe_3O(SO_4)_6(OH)_3]^{5\ominus}, [Fe_3O(O_2CCH_3)_6(OH_2)_3]^{\oplus},$$

$$[Fe_3O(O_4C_2)_6(OH_2)_3]^{5\ominus,} [Fe(C_4H_4O_6)_2]^{2\ominus/\ominus}, Fe(C_4H_4O_6),$$

$Fe_2(C_4H_4O_6)_3$, $Fe(C_3H_5O_3)_2$, $Fe(C_6H_{11}O_7)_2$ , $[Fe(C_2O_4)_3]^{3\ominus}$ ,

$FeC_2O_4$, $[Fe(C_2O_4)_2]^{2\ominus}$, $Fe(acac)_3$, $Fe(acac)_2$, $Fe(C_6H_6O_7)$,

$Fe(C_6H_5O_7)$.

weitere ausschließlich oder überwiegend O-haltige Liganden für Fe(II) oder Fe(III) können aber auch mehrfache cyclische Ether wie Sphäranden, Cryptanden, Cryptasphäranden, Hemisphäranden, Coronanden oder offenkettige Vertreter dieser Ether sowie Podanden sein.

Weiter können verwendet werden Komplexe mit N-haltigen Chelat-Liganden wie Ethylendiamin(en), 1,10-Phenanthrolin(phen), 1,8-Naphthpyridin(napy), 2,2'-Bipyridin (bipy) und Dibenzo[b,i]-1,4,8,11-tetraaza-(14)annulen (taa), d.h. Verbindungen wie

$[Fe(en)(H_2O)_4]^{2\oplus/3\oplus}$, $[Fe(en)_2(H_2O)_2]^{2\oplus/3\oplus}$,

$[Fe(en)_3]^{2\oplus/3\oplus}$, $[Fe(phen)_3]^{2\oplus/3\oplus}$, $[Fe(napy)_4]^{2\oplus/3\oplus}$,

$[Fe(bipy)_4]^{2\oplus/3\oplus}$ und

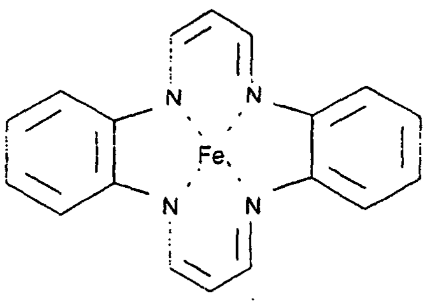

(Fe(taa)),

aber auch Komplexe des Eisens mit verschiedenen, substituierten. Porphyrinliganden, wie sie aus der Literatur bekannt sind (beispielsweise B. Mennier, Chem. Rev., Vol 92 (8), S. 1411-1456, 1992). Andere N-haltige Liganden sind das Phthalocyanin und Derivate davon, wie

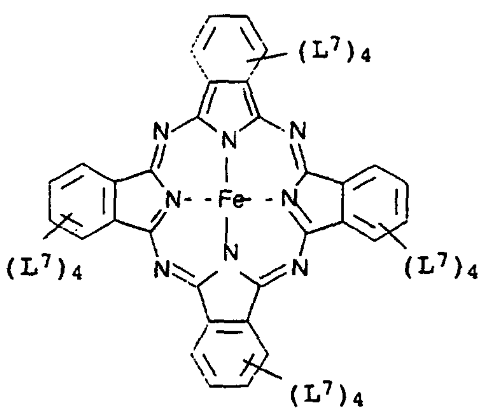

und

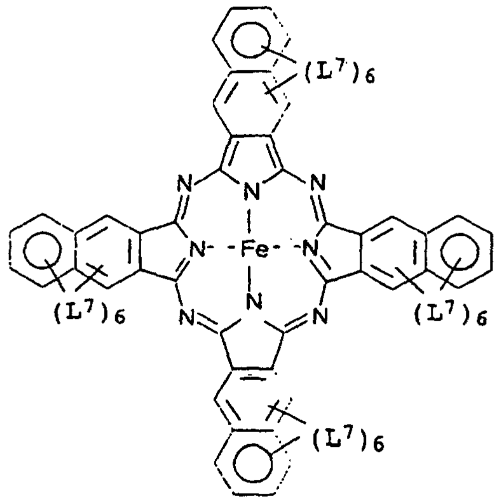

Die Reste $L^7$ können unabhänig voneinander bedeuten Wasserstoff, Halogen, $SO_3H$, $SO_2NH_2$, $SO_2NH(C_1\text{-}C_{12}\text{-Alkyl})$, $SO_2N(C_1\text{-}C_{12}\text{-Alkyl})_2$, $CONH_2$, $CONH(C_1\text{-}C_{12}\text{-Alkyl})$, $CON(C_1\text{-}C_{12}\text{-Alkyl})_2$, Cyano, Hydroxy, $C_1$-$C_{12}$-Alkyl, $C_1$-$C_{12}$-Alkoxy oder $C_1$-$C_{12}$-Alkylthio. Bevorzugte Halogene sind Cl und Br.

Mit N,O-haltigen Liganden, wie Ethylendiamintetraessigsäure (EDTA) oder Nitrilotriessigsäure (NTA), ergeben sich Verbindungen wie

$$[Fe(EDTA)(H_2O)]^{\ominus/2\ominus}, [Fe(NTA)(H_2O)_2] \text{ bzw. } [Fe(NTA)(H_2O)_2]^{\ominus},$$

mit 8-Hydroxychinolin(chin) oder 5-Methyl-8-hydroxychinolin ($H_3C$chin) Verbindungen wie

$$[Fe(chin)_3]/[Fe(chin)_3]^{2\ominus} \text{ bzw.}$$

$$[Fe(H_3C\text{-chin})_3]/[Fe(H_3C\text{-chin})_3]^{2\ominus},$$

welche sich ebenfalls verwenden lassen.

Weitere erfindungsgemäß einzusetzende Fe-Verbindungen sind Fe-Komplexe mit Schiff-Basen von Salicylaldehyden.

Die Herstellung dieser N,O-haltigen Liganden ist bekannt und erfolgt in der Regel durch Kondensation von aromatischen oder heteroaromatischen $\alpha$-Hydroxyaldehyden mit einem aliphatischen oder aromatischen Diamin oder Mehrfachamin. Anschließend erfolgt die Umsetzung der Liganden mit einem Fe-Salz in wäßriger Lösung.

Andere verwendbare Fe-Verbindungen mit S-haltigen Liganden sind etwa

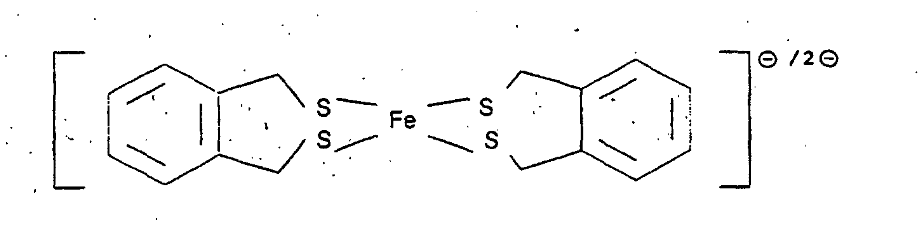

oder $[Fe_4S_4(SR)_4]^{4\ominus/3\ominus}$, aber auch Komplexe des Fe(II)/Fe(III) mit Dithiocarbamaten $R_2NCS_2^{\ominus}$ wie etwa $[Fe(S_2CNR_2)_3]^{\ominus}$ ($R=CH_3$, $C_2H_5$).

Weiter lassen sich auch Verbindungen der Gruppe e) einsetzen. Bevorzugt werden bei den Fe-Halogeniden die Fe(II)- und Fe(III)-Salze von Cl und Br, sowie die Komplexverbindungen $FeX_4^{\ominus/2\ominus}$ (X=Cl,Br) eingesetzt. Zu den erfindungsgemäß einzusetzenden Fe-Pseudohalogenid-Verbindungen zählen beispielsweise $[Fe(CN)_6]^{3\ominus}$/[Fe

$(CN)_6]^{4\ominus}$ sowie Thiocyanatkomplexe der Reihe $[Fe(SCN)_{3-X}(H_2O)_{3+X}]^{X\oplus}$ (x = 0, 1, 2).

Als Gegenionen aller aufgeführten negativ geladenen Komplexionen finden bevorzugt $H^{\oplus}$, $Na^{\oplus}$, $K^{\oplus}$ und Ammoniumionen $NH_4^{\oplus}$ sowie $N(CH_3)_4^{\oplus}$, bei den Hexacyanoferraten aber neben $K^{\oplus}$ auch $Fe^{2\oplus}$ im Falle des $[Fe(CN)_6]^{3\ominus}$ und $Fe^{3\oplus}$ im Falle des $[Fe(CN_6)]^{4\ominus}$ Einsatz.

Bei den aufgeführten positiv geladenen Komplexionen werden bevorzugt als Gegenionen $Cl^{\ominus}$, $Br^{\ominus}$, $I^{\ominus}$, $SO_4^{2\ominus}$, $H_3CCO_2^{\ominus}$, $CrO_4^{2\ominus}$, $BF_4^{\ominus}$ sowie $B(C_6H_5)_4^{\ominus}$ eingesetzt.

Gemäß einer weiteren bevorzugten Ausführungsform des erfindungsgemäßen Verfahrens wird der Teilstrom der Lösung des Stabilisatorsystems vor der Rückführung mit Sauerstoff behandelt. Die Behandlung mit Sauerstoff kann bei einer Temperatur von 20 bis 200°C, vorzugsweise 50 bis 170°C und insbesondere 100 bis 150°C, vorgenommen werden. Die Behandlung mit Sauerstoff kann mit Vorteil mit einem sauerstoffhaltigen Gasgemisch vorgenommen werden, insbesondere einem im Wesentlichen aus Sauerstoff und Stickstoff bestehendem Gasgemisch, wobei dessen Sauerstoffgehalt 3 bis 10 Vol.-% beträgt. Ein geeignetes sauerstoffhaltiges Gasgemisch ist z.B. sogenannte Magerluft. Die Behandlung kann bei Normaldruck oder überatmosphärischem Druck erfolgen. Die Behandlung mit Sauerstoff führt zu einer effektiven Regenerierung freier N-Oxyl-Radikale.

Zur Durchführung der Sauerstoffbehandlung sind alle Vorrichtungen geeignet, die das Inkontaktbringen einer Flüssigkeit, insbesondere einer viskosen Flüssigkeit, mit einem Gas gestatten, z.B. Vorrichtungen zum Durchperlen einer Flüssigkeit mit Gas, zum Einpressen eines Gasstroms in einen Flüssigkeitsstrom usw. Es können auch geeignete Mischbehälter, z.B. gerührte Mischbehälter, vorgesehen werden. Eine Anlage, die zur Durchführung des erfindungsgemäßen Verfahrens mit Sauerstoffbehandlung geeignet ist, ist in Fig. 2 dargestellt.

Die Erfindung wird nun durch die nachfolgenden Beispiele näher veranschaulicht.

Beispiel 1 und Vergleichsbeispiel 1

Rohstyrol der nachstehenden Zusammensetzung wurde mit einer Rate von 100 kg/h in einer Destillationsanordnung destilliert, wie sie in Figur 1 dargestellt ist. Als Konzentrator 4 fand ein Dünnfilmverdampfer Verwendung. Zum Zulauf der 1. Kolonne dosierte man eine Lösung des nachstehenden N-Oxyl-Radikals in Rohstyrol, so daß die Konzentration an N-Oxyl-Radikalen im Sumpf der 1. Kolonne stets im Bereich von 5 bis 100 mg/kg lag .

Rohstyrol-Zusammensetzung:

1 Gew.-% Benzol,
2 Gew.-% Toluol,
40 Gew.-% Ethylbenzol,
56 Gew.-% Styrol
1 Gew.-% Hochsieder.

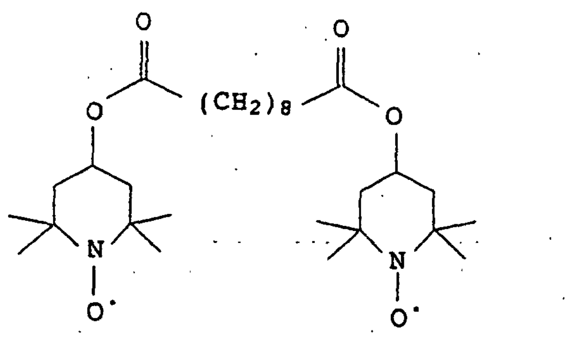

Der Hochsiederanteil aus dem Dünnfilmverdampfer wurde entweder verworfen (Vergleichsbeispiel 1) bzw. ein Teilstrom von 65% des Hochsiederanteils wurde in die Ethylbenzolkolonne 2 zurückgeführt (Beispiel 1). In der nachstehenden Tabelle sind die Viskositätswerte und der Styrol- und Polymergehalt des Hochsiederanteils aus dem Dünnfilmverdampfer angegeben. Die Viskosität wurde mit dem Viscotester VT 02 der Fa. Haake Meßtechnik, Karlsruhe, Deutschland, gemessen (Nenndrehzahl 62,5 $min^{-1}$; Drehkörper 3). Der Polymergehalt wurde entsprechend der ASTM D 2121-95 ermittelt.

Es zeigt sich, daß bei der erfindungsgemäßen Rückführung des Hochsiederanteils die Viskositäten und Polymergehalte auf einem stabilen, wünschenswert niedrigen Niveau gehalten werden können. In Beispiel 1 mit erfindungemäßer Rückführung des Hochsiederanteils werden gegenüber dem Vergleichsbeispiel ohne Rückführung etwa 15% N-Oxyl-Stabilisator eingespart.

Tabelle 1

| | Beispiel 1 | | | | | Vergleichsbeispiel 1 | | | | |
|---|---|---|---|---|---|---|---|---|---|---|
| Tage | mPa s | | | Styr. | Poly | mPa s | | | Styr. | Poly |
| | 60°C | 80°C | 100°C | % | % | 60°C | 80°C | 100°C | % | % |
| Beginn des Beobach-tungszeitraumes | 400 | 220 | 180 | 9,7 | 5 | 540 | 280 | 150 | 7,7 | - |
| + 36 | 800 | 380 | 210 | 3,3 | 5 | 625 | 310 | 175 | 4,7 | - |
| + 85 | 600 | 270 | 170 | 9,4 | 6 | 770 | 370 | 220 | 3,9 | - |
| + 106 | 630 | 280 | 140 | 8,7 | 4 | 800 | 410 | 250 | 2,6 | - |
| + 127 | 780 | 330 | 190 | 9,7 | 6 | 1300 | 800 | 475 | 2,7 | - |
| + 157 | 650 | 330 | 190 | 8,8 | - | 1250 | 700 | 450 | 2,9 | - |
| + 197 | 380 | 190 | 120 | 9,4 | - | 600 | 340 | 200 | 2,8 | - |

Beispiel 2 und Vergleichsbeispiel 2

Beispiel 1 wurde wiederholt, wobei jedoch als Stabilisator ein N-Oxyl-Radikal der nachstehenden Formel in Verbindung mit einer Eisenverbindung in Form von Eisen-dibenzo[b,i]-1,4,8,11-tetraaza-(14)annulen als Aktivator eingesetzt wurde. Das N-Oxyl-Radikale und die Eisenverbindung wurden in einem Gewichtsverhältnis von 99,9 zu 0,1 verwendet.

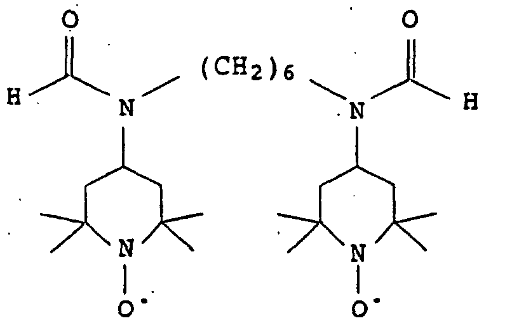

Die Viskositäten und Styrol- und Polymergehalte im Hochsiederanteil aus dem Dünnfilmverdampfer sind in der nachstehenden Tabelle 2 angegeben.

Tabelle 2

| | Beispiel 2 | | | | | Vergleichsbeispiel 2 | | | | |
|---|---|---|---|---|---|---|---|---|---|---|
| Tage | mPa s | | | Styr. | Poly | mPa s | | | Styr. | Poly |
| | 60°C | 80°C | 100°C | % | % | 60°C | 80°C | 100°C | % | % |
| Beginn des Be-obachtungszeitraumes | 1050 | 650 | 450 | 6,7 | 6 | 900 | 480 | 300 | 4,1 | - |
| + 7 | >1300 | 900 | 550 | 4,5 | 7 | 900 | 475 | 275 | 3,8 | - |
| + 21 | 900 | 440 | 250 | 3,8 | 6 | | | | | - |
| + 28 | 700 | 340 | 210 | 4,6 | 6 | 800 | 380 | 220 | 4,1 | - |
| + 35 | 680 | 320 | 175 | 3,9 | 7 | 600 | 230 | 150 | 4,3 | - |
| + 42 | 550 | 275 | 180 | 3,3 | - | 600 | 250 | 150 | 4,2 | - |
| + 49 | 700 | 330 | 170 | 3,9 | - | 630 | 330 | 170 | 3,4 | - |

Tabelle 2 (fortgesetzt)

| | Beispiel 2 | | | | | Vergleichsbeispiel 2 | | | | |
|---|---|---|---|---|---|---|---|---|---|---|
| Tage | mPa s | | | Styr. | Poly | mPa s | | | Styr. | Poly |
| | 60°C | 80°C | 100°C | % | % | 60°C | 80°C | 100°C | % | % |
| + 56 | 700 | 350 | 190 | 3,6 | - | 640 | 330 | 180 | 3,8 | - |
| + 63 | 600 | 320 | 190 | 4,8 | - | 550 | 280 | 180 - | | - |
| + 70 | 300 | 150 | 90 | 5,4 | - | 370 | 180 | 110 | 4,2 | - |
| + 78 | 230 | 130 | 85 | 6,1 | - | 290 | 170 | 90 | 4 | - |
| + 84 | 170 | 90 | 60 | 4,4 | - | 180 | 90 | 60 | 3,8 | - |

Es zeigt sich, daß die Viskositäten und Polymergehalte gegenüber Beispiel 1 weiter gesenkt werden konnten.

Beispiel 3

Vergleichsbeispiel 1 wurde wiederholt, wobei jedoch als Stabilisator ein N-Oxyl der nachstehenden Formel eingesetzt wurde.

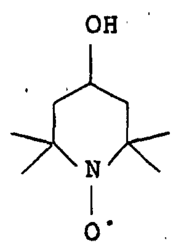

Es wurde eine Probe der am Dünnfilmverdampfer anfallenden Hochsiederfraktion genommen und ESR-spektroskopisch untersucht. Die Messungen wurden mit einem ESR-Spektrometer Miniscope MS 100 in Laborausführung der Fa. Magnettech GmbH, Berlin, Deutschland, durchgeführt. Das Gerät wurde zuvor mit Eichlösungen bekannter Konzentrationen an N-Oxyl-Radikalen kalibriert.

Die Probe wurde 1 h lang auf 140°C erwärmt und erneut ESR-spektroskopisch vermessen. Der berechnete Gehalt an aktivem N-Oxyl-Radikal ist in der nachstehenden Tabelle 3 angegeben.

Tabelle 3

| Probe | vor dem Erwärmen | nach dem Erwärmen |
|---|---|---|
| mg N-Oxyl-Radikal | 436 | 660 |

Die Ergebnisse in Tabelle 3 zeigen deutlich, daß durch Erwärmen auf eine Temperatur von mehr als 130°C die im Hochsiederanteil enthaltenen N-Oxyl-Radikale aktiviert werden können.

**Patentansprüche**

**1.** Verfahren zur kontinuierlichen Gewinnung von Styrol aus einem styrolhaltigen Gemisch durch Destillation des Gemisches in einer Kaskade von n Destillationskolonnen, wobei n für eine positive ganze zahl ≥ 2 steht, wobei

(i) in die erste Kolonne ein ein Stabilisatorsystem enthaltender Zulauf eingespeist wird und/oder in wenigstens eine der n-ten Destillationskolonne vorgeschalteten Destillationskolonne ein Stabilisatorsystem gegeben wird, wobei das Stabilisatorsystem N-Oxyl-Radikale umfasst;

(ii) sich im Sumpf der n-ten Destillationskolonne eine das Stabilisatorsystem enthaltende höher als Styrol siedende Hochsiederfraktion anreichert;

(iii)ein Teilstrom der Hochsiederfraktion zurückgeführt und in wenigstens eine der n-ten Destillationskolonne vorgeschalteten Destillationskolonne gegeben wird;

(iv) die Restmenge der Hochsiederfraktion aus dem Verfahren ausgeleitet wird.

**2.** Verfahren nach Anspruch 1, **dadurch gekennzeichnet, daß** es sich bei der Bochsiederfraktion um die höhersiedenden Anteile des styrolhaltigen Gemisches und/oder Styrololigomere handelt.

**3.** Verfahren nach einem der vorhergehenden Ansprüche, **dadurch gekennzeichnet, dass** die Hochsiederfraktion vor der Rückführung aufkonzentriert wird.

**4.** Verfahren nach einem der vorhergehenden Ansprüche, **dadurch gekennzeichnet, daß** die N-Oxyl-Radikale die (n-1)-te Destillationskolonne im Durchschnitt mindestens 1,4 mal durchlaufen.

**5.** Verfahren nach einem der vorhergehenden Ansprüche, **dadurch gekennzeichnet, daß** der Teilstrom vor der Rückführung auf eine Temperatur von mehr als 130°C erwärmt wird.

**6.** Verfahren nach einem der vorhergehenden Ansprüche, **dadurch gekennzeichnet, daß** das Stabilisatorsystem ferner einen Polymerisationsverzögerer enthält.

**7.** Verfahren nach Anspruch 6, **dadurch gekennzeichnet, daß** es sich bei dem Polymerisationsverzögerer um eine aromatische Nitroverbindung handelt.

**8.** Verfahren nach einem der vorhergehenden Ansprüche, **dadurch gekennzeichnet, daß** das Stabilisatorsystem ferner einen Aktivator enthält.

**9.** Verfahren nach Anspruch 8, **dadurch gekennzeichnet, daß** es sich bei dem Aktivator um eine Eisenverbindung handelt.

**10.** Verfahren nach einem der vorhergehenden Ansprüche, **dadurch gekennzeichnet, daß** der Teilstrom vor der Rückführung mit Sauerstoff behandelt wird.

## Claims

**1.** A process for the continuous recovery of styrene from a styrene-containing mixture by distillation of the mixture in a cascade of n distillation columns, with n being a positive integer ≥ 2, where

(i) a feed stream comprising a stabilizer system is fed into the first column and/or a stabilizer system is introduced into at least one distillation column upstream of the nth distillation column, where the stabilizer system comprises N-oxyl radicals;

(ii)a high boiler fraction having a boiling point higher than that of styrene and comprising the stabilizer system accumulates in the bottom of the nth distillation column;

(iii) a substream of the high boiler fraction is recirculated and introduced into at least one distillation column upstream of the nth distillation column;

(iv) the remainder of the high boiler fraction is discharged from the process.

**2.** A process as claimed in claim 1, wherein the high boiler fraction comprises the relatively high-boiling components of the styrene-containing mixture and/or styrene oligomers.

**3.** A process as claimed in either of the preceding claims, wherein the high boiler fraction is concentrated prior to recirculation. '

**4.** A process as claimed in any of the preceding claims, wherein the N-oxyl radicals pass through the (n-1)th distillation column an average of at least 1.4 times.

5. A process as claimed in any of the preceding claims, wherein the substream is heated to above 130°C prior to recirculation.

6. A process as claimed in any of the preceding claims, wherein the stabilizer system further comprises a polymerization retarder.

7. A process as claimed in claim 6, wherein the polymerization retarder is an aromatic nitro compound.

8. A process as claimed in any of the preceding claims, wherein the stabilizer system further comprises an activator.

9. A process as claimed in claim 8, wherein the activator is an iron compound.

10. A process as claimed in any of the preceding claims, wherein the substream is treated with oxygen prior to recirculation.

**Revendications**

1. Procédé de préparation en continu de styrène à partir d'un mélange contenant du styrène par distillation du mélange dans une cascade de n colonnes de distillation, n représentant un nombre entier positif ≥ 2, et dans lequel

(i) dans la première colonne, on introduit une charge contenant un système stabilisant et/ou, dans au moins une des colonnes de distillation installées en amont de la n-ième colonne de distillation, on introduit un système stabilisant, le système stabilisant contenant des radicaux N-oxyle,
(ii) dans le fond de la n-ième colonne de distillation se concentre une fraction lourde d'un point d'ébullition supérieur à celui du styrène et contenant le système stabilisant,
(iii) un courant partiel de la fraction lourde est recyclé et envoyé dans au moins une colonne de distillation installée en amont de la n-ième colonne de distillation,
(iv) le reste de la fraction lourde est évacué du processus.

2. Procédé selon la revendication 1, **caractérisé en ce que** la fraction lourde est constituée des composants à plus haut point d'ébullition du mélange contenant le styrène et/ou d'oligomères de styrène.

3. Procédé selon l'une des revendications qui précèdent, **caractérisé en ce que** la fraction lourde est concentrée avant d'être recyclée.

4. Procédé selon l'une des revendications qui précèdent, **caractérisé en ce que** les radicaux N-oxyle passent en moyenne au moins 1,4 fois dans la (n-1)ème colonne de distillation.

5. Procédé selon l'une des revendications qui précèdent, **caractérisé en ce que** le courant partiel est, avant son recyclage, chauffé à une température de plus de 130°C.

6. Procédé selon l'une des revendications qui précèdent, **caractérisé en ce que** le système stabilisant contient en outre un ralentisseur de polymérisation.

7. Procédé selon la revendication 6, **caractérisé en ce que** le ralentisseur de polymérisation est un composé aromatique nitré.

8. Procédé selon l'une des revendications qui précèdent, **caractérisé en ce que** le système stabilisant contient en outre un activateur.

9. Procédé selon la revendication 8, **caractérisé en ce que** l'activateur est un composé de fer.

10. Procédé selon l'une des revendications qui précèdent, **caractérisé en ce que** le courant partiel est traité par de l'oxygène avant son recyclage.

Fig. 1

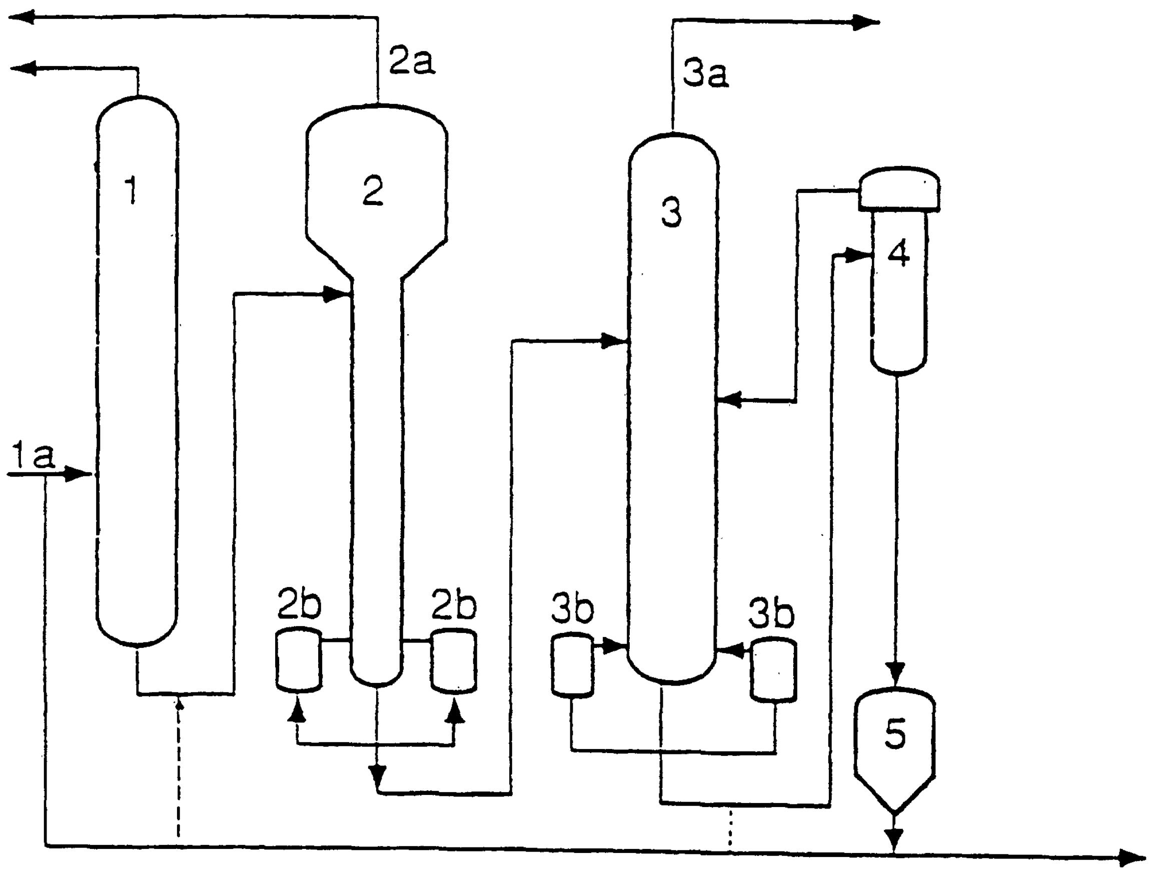

Fig. 2